Europäisches Patentamt

(19) European Patent Office     (11) Publication number:     **0 313 175**
**B1**
Office européen des brevets

(12)                          EUROPEAN PATENT SPECIFICATION

(45) Date of publication of the patent specification:        (51) Int. Cl.⁵: **A61F 5/448**
   **13.06.90**

(21) Application number: **88202885.5**

(22) Date of filing: **24.07.87**

(60) Publication number of the earlier application in accordance
   with Art. 76 EPC: **0255310**

(54) **Ostomy coupling.**

(30) Priority: **31.07.86 GB 8618693**                    (73) Proprietor: **E.R. Squibb & Sons, Inc.,**
           **13.08.86 GB 8619716**                         **Lawrenceville-Princeton Road, Princeton,**
                                                            **N.J. 08540-4000(US)**

(43) Date of publication of application:
   **26.04.89 Bulletin 89/17**                          (72) Inventor: **Steer, Peter Leslie, Kingscote Woodlands**
                                                            **Rise, East Grinstead Sussex(GB)**
                                                            Inventor: **Wiltshire, Neil Phillip, 1 Ormus Cottages**
(45) Publication of the grant of the patent:                **Newchapel Road, Lingfield Surrey(GB)**
   **13.06.90 Bulletin 90/24**

                                                         (74) Representative: **Cook, Anthony John et al, D. YOUNG &**
(84) Designated Contracting States:                         **CO. 10, Staple Inn, London, WC1V 7RD(GB)**
   **AT BE CH DE ES FR GR IT LI LU NL SE**

(56) References cited:
   **EP-A- 0 135 269**
   **EP-A- 0 163 979**
   **WO-A-85/03427**
   **GB-A- 1 568 860**
   **GB-A- 1 579 875**
   **GB-A- 2 121 902**

ACTORUM AG

## Description

The present invention relates to a coupling for releasibly connecting a bag or pouch for receiving discharged waste to a pad. The pad is attached to the body of a wearer. Such bags are often called ostomy bags.

As is explained in UK Patent No. 2 121 902 desirable features of an ostomy coupling are that it is easily coupled and uncoupled for changing or emptying of the bag, has good security of attachment and sealing, and has a flat design so that the bag is unobtrusive when worn under light garments or sports clothing. It has been proposed that an ostomate should wear a pad of medical/surgical adhesive material surrounding his stoma and that an ostomy bag or pouch should be connected thereto. In British Patent Numbers 1 021 145 and 1 099 455 there are proposals for achieving this. A simple coupling using relative rotation of its two parts for connection and disconnection is disclosed in British Patent No. 1 579 875.

According to the present invention, there is provided a three-part ostomy coupling in which rotation of a cam ring to one position prevents the disengagement of two parts of the coupling and to another position allows the parts to be sprung apart manually.

The invention will be better understood from the following non-limiting description of examples thereof given with reference to the accompanying drawings in which:-

FIG. 1 shows in cross-sectional view one example of a first part which is preferably but not necessarily a body side coupling element;

FIG. 2 is a similar view of a second part usable in an example of the invention;

FIG. 3 is a similar view of a third part, the common axis of the stomal aperture being shown in these figures as a vertical chain-dotted line; and

FIGS. 4 and 5 are respectively diagrammatic views mutually at right angles of an assembled coupling according to the invention, FIG. 4 being a cross-section on the line A-A seen in FIG. 5.

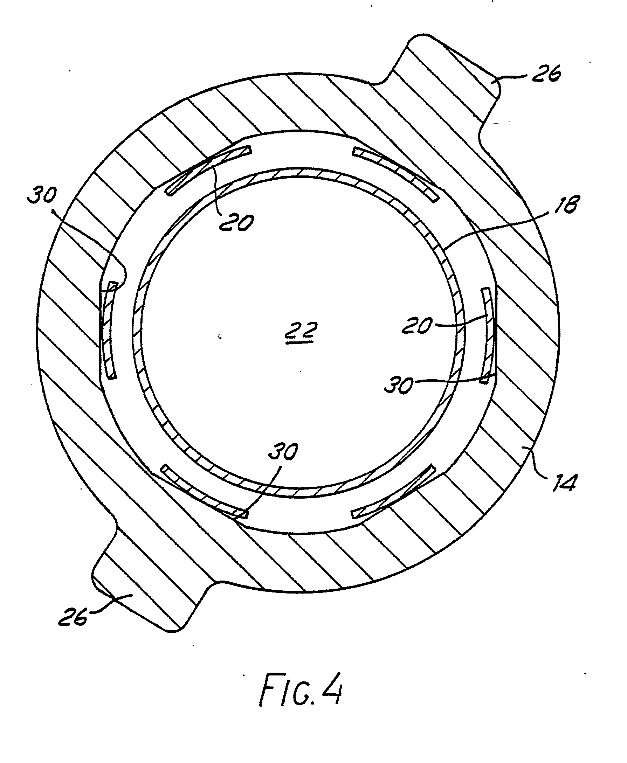

The illustrated ostomy coupling includes a first coupling element 12, a second coupling element 10, and a locking ring 14 in the form of a cam ring. The second coupling element 10 is the body side member and has a flange 16, a central chute 18, and a series of spaced projections 20 surrounding the chute 18. Preferably, the second coupling element 10 is made in one piece as a molding of plastic. The projections 20 are arranged to surround the chute 18 and are arcuate in form (see FIG. 4). The projections 20 are spaced from one another in a peripheral direction by a distance greater than, preferably sightly greater than, the arcuate extent of each projection 20. Preferably, the projections 20 are all of equal arcuate length, but this is not essential. The chute 18 is shown as cylindrical surrounding the stomal orifice 22, but minor departures from circularity are considered to be within the scope of the present invention.

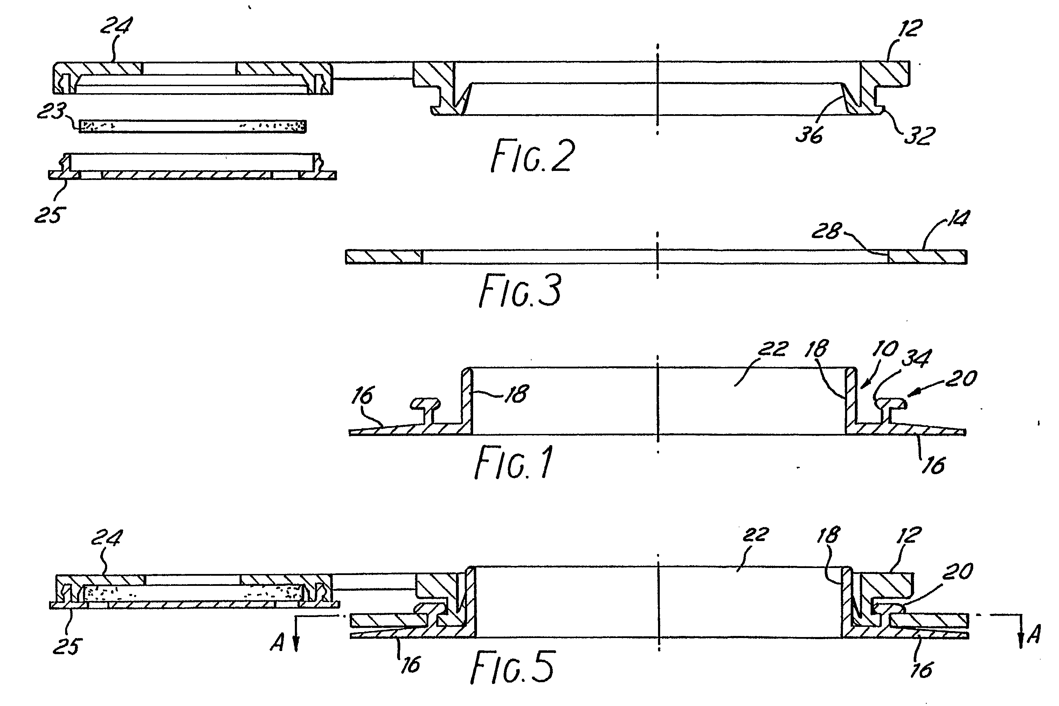

The first element 12 of the coupling illustrated in FIG. 2 is the bag side coupling element 12. In this embodiment of the invention it is similar to the one described and illustrated in British Patent No. 2 121 902, FIG. 5, to which the reader is referred for a detailed description. The bag side coupling element 12 includes a filter housing 24. This may take the form shown in U.K. Patent Application Publication No. 2177926. The housing 24 is not essential to the present invention. The advantages of locking the two coupling elements 10, 12 together in the manner described are also obtained in ostomy couplings which do not have a filter housing. The first coupling element 12 includes a flexible seal strip 36. This part 36 on the first coupling element 12 is a flexible and deflectable sealing means, and may be of the kind disclosed and illustrated in British Patent No. 1568860.

The cam ring 14 is a plain flat ring having ears 26, as shown in FIG. 4, and having a central hole therein bounded by a wall 28 which is part circular with the part circular portions being joined by straight portions 30, as shown in FIG. 4. These straight portions 30, in effect, act as cams and in one rotary position of the ring 14, namely the position shown in FIG. 4 prevent the projections 20 from being forced radially outwardly and in another rotary position, that is to say in a position where the flat portions 30 are located in the spaces between the projections 20, the restraint against outward movement of the projections 20 is removed.

It will be understood from this that the function of the ring 14 is to provide a positive locking, and the lock position (to which it is moved by the wearer of the bag gripping the ears 26 and twisting about the axis of the stomal orifice) is that shown in FIG. 4. In the unlocked position of the ring 14, the first and second parts 10, 12 of the coupling can be separated from each other manually by an axial pull which results in a slight deformation of the projections 20 permitting the rim 32 to spring past the inwardly extending parts 34 of the respective projection.

It has been previously suggested, for example in British Patent Specifications Nos. 1021145 and 1579875, to employ a relative rotation to effect a locking between two parts of an ostomy coupling. It is believed that neither of these prior proposals was effective in practice because of difficulty in operation and unreliability of sealing against egress of liquid. On the other hand, the invention disclosed herein embodies well tried techniques which have proven successful, together with the valuable extra feature of a cam ring or locking ring which provides a positive assurance that the parts of the coupling cannot be separated except when desired by the wearer.

## Claims

1. A three-part ostomy coupling in which rotation of a cam ring (14) to one position prevents the disengagement of two parts (10, 12) of the coupling and to another position allows the parts to be sprung apart manually.

2. A coupling according to claim 1 in which the said two parts are of resilient plastics material and consist of a first part (12) forming a wall around a stomal orifice and having an outwardly projecting rim (32) extending from the wall way from the stomal orifice and a second part (10) which includes walls (20) each having an inwardly projecting portion (34) which can be sprung past the rim (32) due to the resilience to separate the two parts of the coupling.

3. A coupling according to claim 2 in which the second part (10) has a laterally extending flange (16) from which the walls project.

4. A coupling according to any of claims 1 to 3 in which the cam ring (14) has a cam surface (28) formed by cylindrical surfaces alternating with flat surfaces (30).

## Patentansprüche

1. Dreiteilige Ostomiekupplung, in der durch Drehen eines Nockenrings (14) in eine Stellung, das Lösen der zwei Teile (10, 12) der Kupplung verhindert wird, und das Drehen in eine andere Stellung ermöglicht, daß die Teile manuell getrennt werden können.

2. Kupplung nach Anspruch 1, wobei die beiden Teile aus elastischem Kunststoffmaterial sind und sich aus einem ersten Teil (12), das eine eine Stomaöffnung umgebende Wand bildet und einen nach außen vorspringenden Rand (32) aufweist, der sich von der Wand weg von der Stomaöffnung erstreckt, und aus einem zweiten Teil (10) zusammensetzen, das Wände (20) aufweist, die jeweils einen nach innen vorspringenden Abschnitt (34) haben, der zum Trennen der beiden Teile der Kupplung infolge der Verbiegbarkeit über den Rand (32) schnappen kann.

3. Kupplung nach Anspruch 2, wobei das zweite Teil (10) einen sich seitlich erstreckenden Flansch (16) aufweist, von dem die Wände vorspringen.

4. Kupplung nach einem der Ansprüche 1 bis 3, wobei der Nockenring (14) eine Nockenfläche (28) aufweist, die durch zylindrische Flächen abwechselnd mit flachen Flächen (30) gebildet wird.

## Revendications

1. Raccord de stomie en trois parties, dans lequel une bague de came (14), en tournant dans une position, empêche deux parties (10, 12) du raccord de se séparer, et, en tournant dans une autre position, permet aux parties d'être séparées manuellement de manière élastique.

2. Raccord selon la revendication 1, dans lequel lesdites deux parties sont en matière plastique élastique et se composent d'une première partie (12) formant une paroi autour d'un anus artificiel et comportant un rebord (32), faisant saillie vers l'extérieur, qui part de la paroi à l'opposé de l'anus artificiel, et une seconde partie (10) comprenant des parois (20) comportant chacune une partie (34) faisant saillie vers l'intérieur, que l'on peut faire passer élastiquement au-delà du rebord (32) pour séparer les deux parties du raccord.

3. Raccord selon la revendication 2, dans lequel la seconde partie (10) comporte une bride (16) dirigée latéralement, dont font saillie les parois.

4. Raccord selon l'une quelconque des revendications 1 à 3, dans lequel la bague de came (14) a une surface de came (28) formée par des surfaces cylindriques alternant avec des surfaces plates (30).

24

23

25

*FIG.2*

12

36    32

28    14

*FIG.3*

22   18   10   34

16                18                    20

16

*FIG.1*

24                          22      18    12

25                                           20

A    16    *FIG.5*    16    A

EP 0 313 175 B1

FIG.4